# EUROPEAN PATENT APPLICATION

(11) **EP 4 696 675 A1**
(43) Date of publication of application: **18.02.2026**
(21) Application number: 24788951.2
(22) Date of filing: 03.04.2024
(51) Int. Cl.: C07C 51/377, C07C 51/43, C07C 57/04, C07B 35/06

(54) **METHOD FOR PRODUCING ACRYLIC ACID THROUGH DEHYDRATION OF LACTIC ACID, AND APPARATUS FOR MANUFACTURING SAME**

(30) Priority: 12.04.2023 KR 20230048056
(71) Applicant: LG CHEM, LTD., Seoul 07336 (KR)
(72) Inventor: HONG, Daeho, Daejeon 34122 (KR); CHOI, Inho, Daejeon 34122 (KR); CHA, Seoncheol, Daejeon 34122 (KR); KANG, Tae Hun, Daejeon 34122 (KR); LIM, Joonhyung, Daejeon 34122 (KR)
(74) Representative: Goddar, Heinz J.
(86) International application number: PCT/KR2024/004307
(87) International publication number: WO 2024/215008

(57) **Abstract**

Provided are a method for producing an acrylic acid through dehydration of lactic acid, and an apparatus for manufacturing the same.

## Description

### [Technical Field]

### Cross-reference to Related Application

The present application is based on, and claims priority from, Korean Patent Application No. 10-2023-0048056, filed on April 12, 2023, the disclosure of which is hereby incorporated by reference herein in its entirety.

The present invention relates to a method for producing an acrylic acid through dehydration of lactic acid, and an apparatus for manufacturing the same.

### [Background Art]

An acrylic acid is used as a polymer raw material used in fiber, adhesives, paint, fiber processing, leather, building materials, and the like, and its demand is growing. In addition, the acrylic acid is also used as a raw material of an absorbent resin and is industrially used a lot in absorbent articles such as paper diapers and sanitary napkins, agricultural and horticultural water retaining agents, industrial water stop materials, and the like.

A traditional method of preparing acrylic acid is generally a method of oxidizing propylene in the air. However, since this method is a method of converting propylene into acrolein by a gaseous contact oxidation reaction and subjecting the acrolein to a gaseous contact oxidation reaction to prepare acrylic acid, acetic acid is produced as a by-product, which is difficult to separate from the acrylic acid. In addition, the method of preparing acrylic acid using propylene uses, as a raw material, propylene obtained by refining crude oil which is a fossil resource, and considering issues such as a recent rise in crude oil prices or global warming, etc., the method has a problem in terms of raw material costs or environmental pollution.

In this regard, studies were conducted on a method of preparing acrylic acid from a carbon-neutral biomass raw material. For example, there is a method of preparing acrylic acid (AA) through a gaseous dehydration reaction of lactic acid (LA). This method is generally a method of preparing acrylic acid through an intramolecular dehydration reaction of lactic acid in the presence of a catalyst at a high temperature of 300°C or higher.

However, when acrylic acid is prepared through the dehydration reaction of lactic acid, general measurement methods have a problem of incorrectly measuring the content of lactic acid or taking a long time for analysis, because lactic acid has a high boiling point and forms oligomers. In addition, a by-product propanoic acid forms coke on the catalyst surface, which generates a problem of reducing the catalyst lifespan.

### [Disclosure]

### [Technical Problem]

Accordingly, there is provided a method of preparing an acrylic acid through lactic acid dehydration, which easily controls the reaction process by analyzing reaction products of the lactic acid dehydration in a short period of time.

### [Technical Solution]

Accordingly, there is provided a method of preparing an acrylic acid through lactic acid dehydration, the method including:
the step of preparing reaction products including the acrylic acid through the lactic acid dehydration in the presence of a catalyst (Step 1);
the step of preparing a liquid mixture by condensing the reaction products (Step 2);
the step of analyzing in real time the concentrations of lactic acid, lactic acid derivatives including lactic acid oligomers, acrylic acid, and propanoic acid in the liquid mixture using a near-infrared spectrometer by continuously passing the liquid mixture through an optical cell (Step 3); and
the step of determining any one or more of the reaction temperature of the step 1 and regeneration of the catalyst, based on the analysis results of the step 3 (Step 4).

There is also provided an apparatus for lactic acid dehydration, the apparatus including:
a reaction unit where lactic acid dehydration occurs in the presence of a catalyst; a discharge unit discharging reaction products; an absorption tower or condenser which is connected with the discharge unit to condense the reaction products, thereby preparing a liquid mixture; a supply unit that continuously supplies the liquid mixture from the bottom of the absorption tower or condenser to an optical cell of a near-infrared spectrometer; the near-infrared spectrometer that analyzes in real time the concentrations of lactic acid, lactic acid derivatives including lactic acid oligomers, acrylic acid, and propanoic acid in the supplied liquid mixture; and a near-infrared analysis signal processing and control device.

As used herein, the terms "the first", "the second", and the like are used to describe a variety of components, and these terms are merely employed to differentiate a certain component from other components.

Further, the terms used in this description are just for explaining exemplary embodiments and it is not intended to restrict the present invention.

The singular expression may include the plural expression unless it is differently expressed contextually.

In the present description, the term "include", "equip", or "have" is only used for explaining characteristics taken effect, numbers, steps, components, or combinations thereof, and do not exclude the existence or the possibility of addition of one or more different characteristics, numbers, steps, components or combinations thereof.

Further, in this specification, when a layer or an element is mentioned to be formed "on" or "above" layers or elements, it means that each layer or element is directly formed on the layers or elements, or other layers or elements may be additionally formed between the layers, subjects, or substrates.

The present invention may be variously modified and have various forms, and specific embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

In the present description, the lactic acid may include lactic acid isomers, and the lactic acid derivative is used as a concept encompassing all of naturally occurring lactic acid dimers and lactic acid oligomers.

Hereinafter, the present invention will be described in more detail for better understanding.

The present invention provides a method of preparing an acrylic acid through lactic acid dehydration, the method including: the step of preparing reaction products including the acrylic acid through the lactic acid dehydration in the presence of a catalyst (Step 1); the step of preparing a liquid mixture by condensing the reaction products (Step 2); the step of analyzing in real time the concentrations of lactic acid, lactic acid derivatives including lactic acid oligomers, acrylic acid, and propanoic acid in the liquid mixture using a near-infrared spectrometer by continuously passing the liquid mixture through an optical cell (Step 3); and the step of controlling the preparation temperature of the reaction products and/or determining regeneration of the catalyst, based on the analysis results of the step 3 (Step 4).

Further, the method of preparing an acrylic acid through the lactic acid dehydration may be realized by the following apparatus.

According to one embodiment, provided is an apparatus for lactic acid dehydration, the apparatus including: a reaction unit where lactic acid dehydration occurs in the presence of a catalyst; a discharge unit discharging reaction products; an absorption tower or condenser which is connected with the discharge unit to condense the reaction products, thereby preparing a liquid mixture; a supply unit that continuously supplies the liquid mixture from the bottom of the absorption tower or condenser to an optical cell of a near-infrared spectrometer; the near-infrared spectrometer that analyzes in real time the concentrations of lactic acid, lactic acid derivatives including lactic acid oligomers, acrylic acid and propanoic acid in the supplied liquid mixture; and a near-infrared analysis signal processing and control device.

Lactic acid is often used in the preparation of acrylic acid. When lactic acid is dehydrated to prepare acrylic acid, it proceeds by a gas phase reaction, and therefore, lactic acid needs to be vaporized to lactic acid molecules. At this time, lactic acid form oligomers due to its high boiling point and a high reaction temperature, and when gas chromatography is used, which is generally used for analyzing products, the content of lactic acid in the reaction products tends to be underestimated, and when liquid chromatography is used, the reaction products must be first separated, and thus there is a problem that the analysis of the product content is rather delayed.

As described above, when the content of each component in the reaction products is measured incorrectly or the content analysis is delayed, problems may occur in the reaction process by causing a decrease in the reaction activity of the catalyst due to the formation of coke on the catalyst surface. As a result, it may affect the yield of the desired acrylic acid to cause problems in the economic feasibility of the entire process.

Accordingly, the present inventors found that the above problems may be prevented when the reaction products are condensed to prepare a liquid mixture, which is passed through an optical cell, and a near-infrared spectrometer is used to analyze the concentrations of lactic acid, lactic acid derivatives including lactic acid oligomers, acrylic acid, and propanoic acid, which are the main products in the liquid mixture, in real time during the reaction, and the reactor temperature and/or the regeneration time of the catalyst in the reactor are/is quickly and accurately determined, thereby completing the present invention.

Hereinafter, each step will be described in detail.

### (Step 1)

In the method of preparing an acrylic acid through lactic acid dehydration of the present invention, the step 1 is a step of preparing reaction products including the acrylic acid through the lactic acid dehydration in the presence of a catalyst.

The production reaction of acrylic acid by intramolecular dehydration of lactic acid may proceed as follows, and is generally known to proceed in the presence of a catalyst.

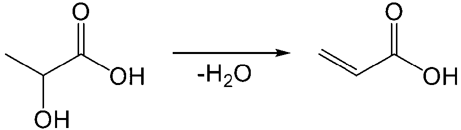

To prepare an acrylic acid through lactic acid dehydration, a step of obtaining lactic acid molecules by vaporizing lactic acid may be generally included, and with regard to the lactic acid vaporization, reactors and/or reaction conditions generally known in the art to which the present invention pertains may be applied.

The method of producing acrylic acid from vaporized lactic acid may also employ catalysts, reactors, and/or reaction conditions generally known in the art to which the present invention pertains.

In addition, the dehydration reaction using a solid catalyst may proceed by a continuous reaction using a fixed reactor, a batch reaction, or the like. When the fixed reactor is used, the product may be continuously prepared by charging the solid catalyst in the reactor and continuously supplying the reactants to the reactor for reaction.

Further, during the lactic acid dehydration, lactic acid may be supplied in the form of an aqueous solution, and at this time, the concentration of the lactic acid aqueous solution may be 10% by weight or more, 20% by weight or more, or 30% by weight or more, and 80% by weight or less, 70% by weight or less, 60% by weight or less, 50% by weight or less, or 40% by weight or less.

The temperature of the dehydration reaction may be about 300°C to about 500°C, or about 320°C to about 400°C. The reaction pressure may be about 1 atm to about 5 atm, or about 1 atm to about 2 atm.

The supply velocity of the reactants may vary depending on the shape or type of reactor, other reaction conditions, etc. Specifically, for example, the gas phase lactic acid supply velocity (Weight Hourly Space Velocity, WHSV) may be advanced to be about 0.05/hr to about 1.0/hr, or about 0.10/hr to about 0.50/hr.

When the supply velocity deviates from the above conditions, the decomposition reaction by hydrogenation proceeds, which may cause a problem that the reaction efficiency is lowered or the conversion rate is lowered.

In the step 1 of the present invention, the reaction products produced through the lactic acid dehydration includes acrylic acid, and may include unreacted lactic acid, lactic acid oligomers, and propanoic acid which is produced by a side reaction. Meanwhile, the lactic acid oligomer refers to a substance in which lactic acid reacts with each other to form a dimer, trimer, etc., or a dehydration condensation form in which water molecules are removed.

### (Step 2)

The step 2 of the present invention is a step of preparing a liquid mixture by condensing the reaction products which are prepared in the step 1. The reaction products discharged from the reactor are in an overheated state, and is generally in the form of a gaseous mixture. Therefore, for transport and storage of the reaction products, it is preferable to prepare the reaction products as a liquid mixture.

The liquid mixture of the step 2 may employ an absorption tower or condenser to be described later, and to the configuration of the absorption tower or condenser, any apparatus and/or conditions generally known in the art to which the present invention pertains may be applied.

Meanwhile, the preparation of the liquid mixture in the step 2 may be carried out under temperature conditions of 50°C to 250°C. When the preparation is carried out at a temperature of higher than 250°C, it is failed to liquefy the gaseous reaction product, and thus the loss may increase, and when carried out at a temperature of lower than 50°C, excessive energy consumption may be a problem. Preferably, the preparation may be carried out at a temperature of 60°C or higher, 70°C or higher, or 80°C or higher, and 220°C or lower, 200°C or lower, or 180°C or lower.

According to an aspect of the present invention, in the step 2, the gaseous products may be prepared into a liquid mixture using water. The method of using water in the absorption tower or condenser is not particularly limited. However, in order to facilitate contact with the reaction products, water may be sprayed by installing a spray nozzle at the top portion of the absorption tower or condenser or at one or more locations thereof. The direction of water spraying may be performed in a direction parallel to the flow direction of the reaction products, or in a direction against the flow direction, or any combination of thereof.

In the step 2 of the present invention, the method of preparing the liquid mixture may employ the above-described condenser or absorption tower, wherein the condenser or absorption tower may be provided in singular or plural form. For example, when the liquid mixture is prepared through a plurality of condensers, a first condenser and a second condenser may be used. In this manner, when the liquid mixture is prepared through a plurality of condensers, there is an effect of simplifying the composition of the condensate, and thus the NIR signal ratio of each component is increased, thereby improving the accuracy of analysis.

More specifically, the reaction products discharged from the reactor are supplied to the first condenser to form a liquid condensate such as unreacted lactic acid, etc. at the bottom portion of the first condenser, which is discharged as a bottom fraction of the first condenser, and components such as light gas and acrylic acid, etc., excluding unreacted lactic acid, may be discharged as a top fraction at the top portion of the first condenser.

The top fraction of the first condenser may be fed to the second condenser. In the second condenser, the reaction products are further condensed and separated into a bottom fraction containing acrylic acid and propanoic acid and a top fraction containing light gas.

As described above, in the method of preparing the liquid mixture through a plurality of condensers or absorption towers, the concentrations of lactic acid and lactic acid derivatives including lactic acid oligomers, which are discharged from the bottom of the first condenser, may be analyzed in the step 3 described later, and the concentration of propanoic acid discharged from the bottom of the second condenser may be analyzed in the step 3 described later.

Meanwhile, when the first condenser and the second condenser are used as above, the preparation of the liquid mixture in the first condenser of the step 2 may be carried out under temperature conditions of 100°C to 250°C. When the preparation is carried out at a temperature of higher than 250°C, the gaseous reaction product may not be liquefied, which may increase the energy required to separate the lactic acid and lactic acid derivatives including lactic acid oligomers in the subsequent purification step. When the preparation is carried out at a temperature of lower than 100°C, the content of acrylic acid in the bottom fraction of the first condenser may increase and thus the recovery rate of acrylic acid may decrease. Preferably, it may be carried out at a temperature of 110°C or higher, 120°C or higher, or 130°C or higher, and 220°C or lower, 200°C or lower, or 180°C or lower.

The preparation of the liquid mixture in the second condenser may be carried out under temperature conditions of 50°C to 150°C. When the preparation is carried out at a temperature of higher than 150°C, the top fraction of the first condenser may not be liquefied, which may increase the loss of acrylic acid product. When the preparation is carried out at a temperature of lower than 50°C, excessive energy consumption may be a problem. Preferably, it may be carried out at a temperature of 60°C or higher, 70°C or higher, or 80°C or higher, and 130°C or lower, 120°C or lower, or 110°C or lower.

### (Step 3)

The step 3 of the present invention is a step of analyzing in real time the concentrations of lactic acid, acrylic acid, and propanoic acid in the liquid mixture using a near-infrared spectrometer by continuously passing the liquid mixture prepared in the step 2 through an optical cell.

As the liquid mixture is continuously passed through the optical cell, a probe inside the optical cell is contacted or interfaced with the liquid mixture stream. The signals detected therefrom are transmitted to the near-infrared spectrometer, thereby obtaining information about each component in the liquid mixture. The resulting information is then sent to the spectrometer by any means, e.g., a wired cable, wireless transmission, etc., and the spectrometer generates a spectrum of absorbance versus wavelength. The generated spectrum allows determining the concentrations of lactic acid, lactic acid derivatives including lactic acid oligomers, acrylic acid, and propanoic acid, which are the main components of the sampled liquid mixture stream. As described, the concentration of each component, i.e., the composition in the liquid mixture may be analyzed in real time by measuring the near-infrared absorbance of the unique wavelength of each component in the liquid mixture using the near-infrared spectrometer. The signals generated based on the information of the liquid mixture analyzed in real time allow controlling any step in the entire process of the present invention.

The near-infrared ray used in the near-infrared spectrometer of the present invention has a wavelength range of 4000 cm⁻¹ to 12500 cm⁻¹. In general, it is known that the near-infrared absorbance for -CH, -OH, -NH functional groups is quite weak, and the absorbance change for a unit concentration change in the near-infrared absorption spectrum is more sensitive than the absorbance change for visible light (12,000 cm⁻¹ to 25,000 cm⁻¹) and mid-infrared light (400 cm⁻¹ to 4,000 cm⁻¹). Therefore, when light in the near-infrared region is used, it is possible to analyze the concentrations of major components without requiring additional treatment such as sample dilution, etc., and quantitative analysis of multiple components is possible at the same time, thereby shortening the analysis time. More preferably, the near-infrared ray at a wavelength range of 5400 cm⁻¹, 6350 cm⁻¹, or 7200 cm⁻¹ to 12500 cm⁻¹ may be used.

The near-infrared spectrometer which may be used in the present invention is not particularly limited, but representative examples thereof may include Bruker and MPA II.

### (Step 4)

The step 4 of the present invention is a step of determining any one or more of the reaction temperature of the step 1 and the catalyst regeneration, based on the analysis results of the step 3.

In the method of preparing acrylic acid through lactic acid dehydration, as the reaction progresses, coking may occur on the catalyst surface due to unreacted products such as lactic acid and lactic acid oligomers. This reduces the surface area of the catalytic active species, and when coke continues to accumulate on the catalyst surface, the generation of the by-product propanoic acid increases, causing loss of acrylic acid during the purification process, making catalyst regeneration impossible, or consuming excessive energy for regeneration. Therefore, in order to secure a conversion rate of the desired lactic acid and a high yield of acrylic acid while continuously operating the step 1 of the present invention, it is important to control the dehydration temperature and to select the catalyst regeneration step at an appropriate time.

According to an aspect of the present invention, as a result of the analysis of the step 3, when the concentration of lactic acid and lactic acid oligomers in the liquid mixture increases by 0.5% by weight to 10% by weight, as compared to the initial reaction conditions, a step of increasing the temperature of the reactor in the step 1 from 1°C to 50°C may be included. As the concentration of lactic acid and lactic acid oligomers increases, the reactor temperature may be further increased to maintain the conversion rate of the reactor at a level equivalent to the initial conditions. In other words, in addition to determining the catalyst regeneration step through the analysis results of the step 3, the reactor temperature may be controlled, thereby reducing the by-product production while controlling the reaction rate. Preferably, when the concentration of lactic acid and lactic acid oligomers in the liquid mixture increases by 1.0% by weight to 9.0% by weight, 1.0% by weight to 8.0% by weight, as compared to the initial reaction conditions, the temperature control of the step 1 may be performed, and the reactor temperature may be raised to 1°C or higher, 2°C or higher, 3°C or higher, 4°C or higher, or 5°C or higher, and 40°C or lower, 30°C or lower, 20°C or lower, or 15°C or lower.

According to one embodiment of the present invention, as a result of the analysis in the step 3, when the concentration of propanoic acid in the liquid mixture is 0.1% by weight to 5% by weight, the catalyst regeneration step may be performed. In order to determine whether or not the catalyst regeneration is performed, the decision may be made by a reference material for determining the catalyst regeneration and comparing with a reference value of the corresponding reference material. In the present invention, it was confirmed that propanoic acid in the liquid mixture is selected as the reference material, and based on the concentration of propanoic acid in the liquid mixture, when the concentration of propanoic acid is 0.1% by weight to 5% by weight, the catalyst regeneration step is performed, which is desirable in terms of ease of the catalyst generation and lifespan. In addition, in the purification process to obtain the final acrylic acid, as the concentration of propanoic acid is higher, it is more difficult to purify acrylic acid. To prevent this, it is preferable to regenerate the catalyst when the concentration of propanoic acid is within the above range. Preferably, the step 4 may be performed when the concentration of propanoic acid in the liquid mixture is 4%, 3%, 2%, 1%, or 0.7% or less.

Meanwhile, the catalyst regeneration step may be performed on the catalyst which is used in the dehydration of lactic acid using a method of removing coke by raising the temperature of the reactor to 320°C to 450°C and flowing a mixed gas containing oxygen at an oxygen concentration of 0.1 mol% to 20 mol%. When the catalyst regeneration temperature is lower than the above, coke regeneration incompletely proceeds, and when the temperature is higher than the above, the catalyst may be overheated and its lifespan may be reduced. In addition, when the oxygen concentration is lower than the above range, the time required for the catalyst regeneration may be delayed, and when the oxygen concentration is higher than the above, the catalyst may be overheated due to the oxidation heat of coke and the lifespan of the catalyst may be reduced. Meanwhile, the mixed gas may include nitrogen, and if necessary, may further include steam. Preferably, the reactor temperature during the catalyst regeneration may be 350°C or higher, 370°C or higher, 380°C or higher, or 390°C or higher, and 430°C or lower, 420°C or lower, or 410°C or lower. In addition, the oxygen concentration of the mixed gas may be 0.5 mol% or more, 1.0 mol% or more, 2.0 mol% or more, or 3.0 mol% or more, and 15 mol% or less, 10 mol% or less, or 7.0 mol% or less.

In addition, the present invention may further include a step of aliquoting a portion of the liquid mixture to perform liquid chromatography analysis. The liquid chromatography analysis has an advantage of being more accurate than other analysis methods. However, the analysis time is usually long, and thus there is a problem that it is difficult to respond immediately through analysis of the reaction products. In contrast, in the present invention, the liquid chromatography analysis is further performed on the same sample, in addition to the near-infrared spectrometry, and the analysis results are directly compared with those of the near-infrared spectrometry to increase reliability of the results of the near-infrared spectrometry.

Further, since the method of preparing acrylic acid of the present invention described above is a continuous process, the compositions of the reactants and reaction products change in real time. Accordingly, based on the composition of the product aliquot measured by liquid chromatography, the composition is compared with the error in the product composition measured in a stationary state using the near-infrared spectrometer, and then, as in the step 3 of the present invention, the composition of the liquid mixture is analyzed using the near-infrared spectrometer in real time, thereby reducing measurement errors. Through this, whether or not an abnormality in the reactor conditions has occurred, such as an increase in the content of the lactic acid derivatives including the lactic acid oligomers and/or an increase in the content of propanoic acid, may be more accurately examined, thereby determining whether or not the step 4 is performed.

Preferably, the measurement errors of the lactic acid and acrylic acid concentrations in the liquid mixture which are measured through the near-infrared spectrometry and liquid chromatography analysis are 1.0% or less, or 0.9% or less. A reference value for determining the error of each component is based on the results of the liquid chromatography analysis. The liquid chromatography is repeatedly performed on the same sample, and the mean value thereof is set as the reference value, which is compared with the result of the near-infrared spectrometry analysis to calculate the error.

Meanwhile, the present invention provides an apparatus for lactic acid dehydration, the apparatus including: a reaction unit 2, 2a, 2b where lactic acid dehydration occurs in the presence of a catalyst; a discharge unit 21, 22 discharging reaction products; an absorption tower or condenser 3, 3a, 3b which is connected with the discharge unit to condense the reaction products, thereby preparing a liquid mixture; a supply unit 31, 33, 35 that continuously supplies the liquid mixture from the bottom of the absorption tower or condenser to an optical cell (NIR Cell) of a near-infrared spectrometer; the near-infrared spectrometer that analyzes in real time the concentrations of lactic acid, lactic acid derivatives including lactic acid oligomers, acrylic acid, and propanoic acid in the supplied liquid mixture; and a near-infrared analysis signal processing and control device 4.

In the apparatus for lactic acid dehydration of the present invention, the catalyst, reaction product, liquid mixture, near-infrared spectrometer , etc. are as described above for reference.

The reaction unit is an area where dehydration of vaporized lactic acid occurs in the presence of a catalyst to produce the reaction products. For example, the reaction unit may be a reactor equipped with a heating unit, and the reactor may include a supply unit to which the reactant lactic acid is supplied, a vaporization unit to vaporize the lactic acid aqueous solution, and a catalyst unit that brings the vaporized lactic acid molecules into contact with the catalyst.

The reaction products prepared in the reaction unit are discharged through the discharge unit which is connected to the reaction unit, and is fed into the absorption tower or condenser which is connected with the discharge unit to condense the reaction products, thereby preparing the liquid mixture. The absorption tower or condenser produces the gaseous reaction products in the form of the liquid mixture.

The shape of the absorption tower or condenser is not particularly limited, but it may be, for example, initiated in a quench or flash vessel, or may include column internals. Further, when the absorption tower is used, water or an organic solvent may be used, and a spray nozzle may be provided to prepare the liquid mixture by bringing the reaction products into contact with water inside the absorption tower. To prevent polymerization of acrylic acid, a polymerization inhibitor may be added to water or organic solvents.

A supply unit is provided at the bottom portion of the absorption tower or condenser to continuously supply the liquid mixture to the optical cell for analysis through the near-infrared spectrometer. The concentration of each component in the liquid mixture that passed through the optical cell is measured using the near-infrared spectrometer described above. The concentration of the product which is analyzed using the near-infrared spectrometer is transmitted as near-infrared analysis signals to the signal processing and control device. The signal processing and control device determines one or more of the reaction temperature in the above-mentioned reaction unit and catalyst regeneration, based on the analysis signals, and controls the reaction temperature by controlling the temperature inside the reactor and/or allows catalyst regeneration by converting the gaseous feed into a mixed gas containing oxygen required for catalyst regeneration.

Further, the absorption tower or condenser may be singular or plural, and specifically, may be one or two. The description of the plurality of absorption towers or condensers is the same as described above.

According to an aspect of the present invention, a liquid chromatography analyzer to which at least a part of the supply unit is branched and connected may be further included. As mentioned above, the near-infrared spectrometry and liquid chromatography analysis are performed at the same time to quickly and accurately analyze the concentration of each component in the liquid mixture.

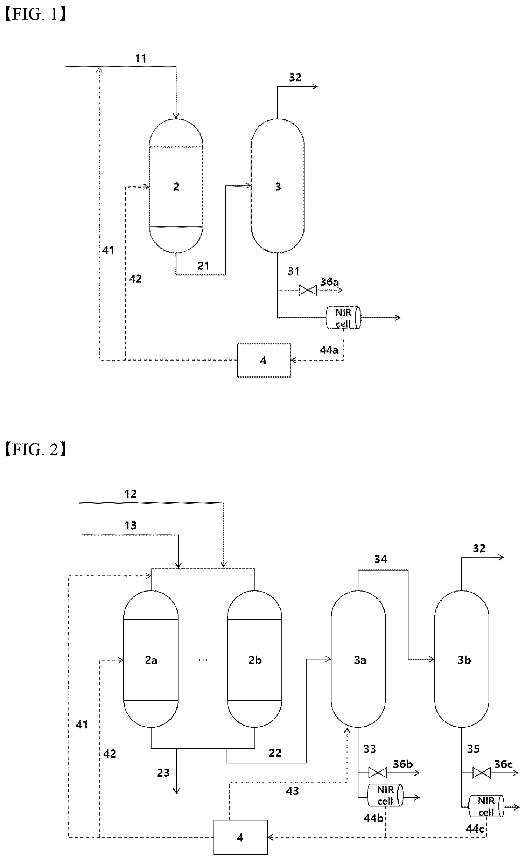

FIG. 1 illustrates a process apparatus which is used in the method of preparing acrylic acid through lactic acid dehydration according to an embodiment of the present invention.

Referring to FIG. 1, a gaseous feed 11 containing a mixture of vaporized high-temperature lactic acid and water vapor, and if necessary, a carrier gas, is introduced into a dehydration reactor 2. The reaction product 21 prepared in the reactor is introduced into a condenser 3, cooled and separated into a liquid mixture 31 containing acrylic acid and a gaseous light product 32. At this time, the composition of the liquid mixture passing through the NIR cell is transmitted to an NIR signal processing and control device 4 at a frequency of twice to three times per minute (44a). When the content of lactic acid and lactic acid derivatives including lactic acid oligomers deviates from the operating range, the temperature of the reactor may be controlled (42) to bring it within the operating range. In general, the catalyst is deactivated over the reaction time, the content of lactic acids increases, and the yield of acrylic acid decreases. Therefore, the conversion rate and the yield of acrylic acid are maintained by increasing the reaction temperature. At the temperature rising above a certain level, when the conversion rate decreases or the content of propanoic acid rises above a certain level, the gaseous feed 11 supplied to the reactor may be switched (41) to put the reactor into the regeneration step. At this time, the composition of the gaseous feed 11 is changed to oxygen and carrier gas, and the temperature of the reactor is also controlled so that the coke accumulated on the catalyst may be oxidized and vaporized. As needed, steam may be further added to the gaseous feed 11 in order to control the heat generated during the oxidation process of coke.

FIG. 2 illustrates a process apparatus using two condensers in the method of preparing acrylic acid through lactic acid dehydration of the present invention.

When condensing is carried out in multiple steps, a lactic acid liquid mixture 33 with a high boiling point is first condensed into a liquid phase in a condenser 3a which is operated at high temperature, and then separated, and the composition is transmitted through an NIR cell into an NIR signal processing and control device 4 (44b). The remaining gaseous light product 34 is separated into the liquid mixture 35 such as acrylic acid, etc. and a light product 32 in a condenser 3b which is operated at low temperature, and the composition of the liquid mixture 35 is transmitted through the NIR cell to the NIR signal processing and control device 4 (44c). Two or more reactors 2a, 2b allows continuous operation; when catalyst regeneration is required (41), regeneration is performed in the corresponding reactor, and the remaining reactors are operated. Two or more condensers 3a, 3b may save energy input in the process of separating lactic acid by controlling the temperature 43 of the condenser 3a which is operated at high temperature. Specifically, when the content of lactic acid products included in the NIR signal 44b which is observed in the lactic acid liquid mixture 33 including lactic acid and lactic acid oligomers increases, or when the content of propanoic acid included in the NIR signal 44c which is observed in the liquid mixture 35 such as acrylic acid, etc. increases, the reactor temperature may be adjusted or the catalyst regeneration may be performed. When the content of acrylic acid product included in the NIR signal 44b which is observed in the lactic acid liquid mixture 33 increases, or when the content of lactic acid products included in the NIR signal 44c which is observed in the liquid mixture 35 such as acrylic acid, etc. increases, the temperature of the condenser 3a operated at high temperature may be adjusted (43).

### [Effect of the Invention]

According to a method and an apparatus of the present invention, each for preparing an acrylic acid through lactic acid dehydration, it is easy to inspect the status of a reactor and to respond to abnormal reactions through real-time continuous measurement of major products.

### [Brief Description of Drawings]

FIG. 1 shows a schematic illustration of a process and an apparatus which are employed in a method of preparing an acrylic acid through lactic acid dehydration according to an embodiment of the present invention; and
FIG. 2 shows a schematic illustration of a process and an apparatus, in which two condensers are employed, in the method of preparing an acrylic acid through lactic acid dehydration according to an embodiment of the present invention.

### [Detailed Description of the Embodiments]

Hereinafter, exemplary embodiments of the present invention will be described in more detail in the following Examples. However, the following Examples are only for illustrating embodiments of the present invention, and thus the content of the present invention is not limited by the following Examples.

### Example 1

Acrylic acid was prepared through lactic acid dehydration according to a process flow chart shown in FIG. 1 and process conditions shown in Tables 1 to 3 below. Table 1 below shows operating conditions of each apparatus during reaction, and Table 2 below shows operating conditions of each apparatus during catalyst regeneration. In addition, Table 3 below shows a method of controlling the composition of a gaseous feed 11 (41) and controlling the temperature of a reactor 2 (42) according to the composition change 44a of a product 31 after condensation.

In detail, as a result of analyzing the liquid product through a NIR cell at the start of the reaction, the content of lactic acid and lactic acid oligomers was 4.0% by weight. The content of lactic acid and lactic acid oligomers in the liquid product increased by 1.3% by weight over time, reaching 5.3% by weight. After confirming that the content of lactic acid and lactic acid oligomers in the liquid product increased, the temperature of the reactor was raised from 352°C to 358°C. Accordingly, it was confirmed that the conversion rate was recovered and the content of lactic acid and lactic acid oligomers was reduced to 4.2% by weight.

Thereafter, after confirming that the content of lactic acid and lactic acid oligomers increased to 6.5% by weight, and the content of propanoic acid increased to 0.6% by weight as the reaction continued, the reaction temperature was raised to 400°C and the composition of the gaseous feed to be introduced was changed to oxygen and nitrogen and the catalyst regeneration step was initiated.

**[Table 1]**

| | Apparatus | | | | | |
|---|---|---|---|---|---|---|
| | 11 | 2 | 21 | 3 | 33 | 34 |
| Temperature (°C) | 25 | 350-360 | 250 | 5.0 | 25 | 5.0 |
| Pressure (bar) | 1.1 | 1.1 | 1.00 | 1.00 | 1.00 | 1.00 |
| Flow rate (g/h) | 45 | 45 | 45 | 45 | 9.0 | 36 |

**[Table 2]**

| | Apparatus | | | | | |
|---|---|---|---|---|---|---|
| | 11 | 2 | 21 | 3 | 31 | 32 |
| Temperature (°C) | 25 | 400 | 350 | 5.0 | 5.0 | 25 |
| Pressure (bar) | 1.2 | 1.2 | 1.00 | 1.00 | 1.00 | 1.00 |
| Flow rate (g/h) | 160 | 160 | 160 | 160 | 135 | 25 |

**[Table 3]**

| Analysis and Control items | Element | Unit | Start of reaction | Before raising temperature | After raising temperature | Before regeneration | Regeneration |
|---|---|---|---|---|---|---|---|
| 44a (31 composition) | Lactic acid | wt% | 2.7 | 2.9 | 2.6 | 3.0 | - |
| | Lactic acid oligomer | wt% | 1.3 | 2.4 | 1.6 | 3.5 | - |
| | Acrylic acid | wt% | 20.0 | 18.9 | 19.7 | 17.8 | - |
| | Propanoic acid | wt% | 0.1 | 0.3 | 0.4 | 0.6 | - |
| 41 (11 composition) | Nitrogen | mol% | 15.0 | 15.0 | 15.0 | 15.0 | 95.0 |
| | Oxygen | mol% | 0.0 | 0.0 | 0.0 | 0.0 | 5.0 |
| | Lactic acid | mol% | 10.0 | 10.0 | 10.0 | 10.0 | 0.0 |
| | Water | mol% | 75.0 | 75.0 | 75.0 | 75.0 | 0.0 |
| 42 (2 temperature) | Temperature | °C | 352 | 352 | 358 | 358 | 400 |

### Example 2

Acrylic acid was prepared through lactic acid dehydration according to a process flow chart shown in FIG. 2 and process conditions shown in Tables 4 to 6 below. Example 2 was operated by adding swing conversion operation of multi-step condensation and reactor to Example 1. Table 4 below shows operating conditions of each apparatus during reaction, and Table 5 below shows the composition of a gaseous feed supplied to the reaction apparatus. In addition, Table 6 below shows a method of controlling gaseous feed 12, 13 and exhaust gas 22, 23 lines to which each reactors 2a, 2b is connected according to the composition changes (44b, 44c) of products 33, 35 after condensation (41), controlling the temperature of the reactor 2 (42), and controlling the temperature of a condenser 3a (43).

In detail, as a result of analyzing, through an NIR cell, the liquid product collected from a first condenser at the start of the reaction, 10.6% by weight, based on the total feed, was collected, in which the content of lactic acid and lactic acid oligomers was 33.3% by weight. In a second condenser, 20.8% by weight of acrylic acid was collected at the start of the reaction. The weight of acrylic acid collected in the second condenser decreased over the reaction time, and when the weight of acrylic acid decreased to 19.5% by weight or less, the condensation temperature of the first condenser was raised by 2°C to 3°C to lower the rate of acrylic acid lost in the first condenser. The amount of the product collected from the first condenser increased over time to 11.4% by weight, based on the total feed, and the content of lactic acid and lactic acid oligomers in the liquid product increased by 6.9% by weight, up to 40.2% by weight. The initial temperature of the first condenser was 125°C, and increased to 130°C before raising the temperature of the reactor. After confirming that the content of lactic acid and lactic acid oligomers in the liquid product increased, the temperature of the reactor was raised from 352°C to 358°C. Accordingly, it was confirmed that the conversion rate was recovered and the amount of the product collected in the first condenser was reduced to 10.5% by weight, based on the total feed, and the content of lactic acid and lactic acid oligomers in the corresponding feed was reduced to 35.4% by weight. At the same time, the temperature of the first condenser was lowered to 127°C.

Thereafter, after confirming that as the reaction continues, the amount of the product collected in the first condenser increased to 13.7% by weight, based on the total feed, the content of lactic acid and lactic acid oligomers in the corresponding feed increased to 41.5% by weight, and the content of propanoic acid in the product collected in the second condenser increased to 0.6% by weight, the connection of reactor 2a was switched from a reaction feed containing lactic acid to a regeneration feed containing oxygen, thereby converting to the exhaust gas in the condenser. Then, the reaction temperature was raised to 400°C and the catalyst regeneration step was initiated. The temperature of the first condenser was controlled by examining the yield of acrylic acid as before raising the temperature of the reactor, and the final temperature of the first condenser just before entering the regeneration step was 133°C.

**[Table 4]**

| | Apparatus | | | | | | | | | | | |
|---|---|---|---|---|---|---|---|---|---|---|---|---|
| | 12 | 13 | 2a | 2b | 22 | 23 | 3a | 33 | 34 | 3b | 32 | 35 |
| Temperature (°C) | 80 | 80 | 350-360 | 400-430 | 250 | 350 | 100-230 | 125-133 | 140 | 80-110 | 122 | 107 |
| Pressure (bar) | 1.5 | 1.5 | 1.5 | 1.5 | 1.2 | 1.2 | 1.1 | 1.1 | 1.1 | 1 | 1 | 1 |
| Flow rate (kg/h) | 4500 | 16000 | 4500 | 16000 | 4500 | 16000 | 4500 | 360 | 4140 | 4140 | 810 | 3330 |

**[Table 5]**

| | | Unit | Content |
|---|---|---|---|
| 12 composition | Nitrogen | mol% | 15.0 |
| | Oxygen | mol% | 0.0 |
| | Lactic acid | mol% | 10.0 |
| | Water | mol% | 75.0 |
| 13 composition | Nitrogen | mol% | 95.0 |
| | Oxygen | mol% | 5.0 |

**[Table 6]**

| Analysis and Control items | Element | Unit | 2a start of reaction | 2a Before raising temperature | 2a After raising temperature | 2a Before regeneration | 2b Start of reaction |
|---|---|---|---|---|---|---|---|
| 44b (33 composition) | Lactic acid | wt% | 21.7 | 20.6 | 21.0 | 17.7 | 21.7 |
| | Lactic acid dimer | wt% | 11.7 | 19.6 | 14.4 | 23.8 | 11.7 |
| | Acrylic acid | wt% | 13.3 | 11.5 | 13.1 | 10.4 | 13.3 |
| | Propanoic acid | wt% | 0.1 | 0.2 | 0.3 | 0.3 | 0.1 |
| 44c (35 composition) | Lactic acid | wt% | 0.5 | 0.6 | 0.4 | 0.7 | 0.5 |
| | Lactic acid dimer | wt% | 0.1 | 0.2 | 0.1 | 0.3 | 0.1 |
| | Acrylic acid | wt% | 20.8 | 19.9 | 20.5 | 19.6 | 20.8 |
| | Propanoic acid | wt% | 0.1 | 0.3 | 0.4 | 0.6 | 0.1 |
| 42 (22 temperature) | Temperature | °C | 352 | 352 | 358 | 358 | 352 |
| 42 (23 temperature) | Temperature | °C | 400 | 410 | 420 | 430 | 400 |
| 43 (33 temperature) | Temperature | °C | 125 | 130 | 127 | 133 | 125 |
| 41 (flow control) | Elements connected | | 12 → 2a → 22, 13 → 2b → 23 | 12 → 2a → 22, 13 → 2b → 23 | 12 → 2a → 22, 13 → 2b → 23 | 12 → 2a → 22, 13 → 2b → 23 | 12 → 2b → 22, 13 → 2a → 23 |

### Experimental Example 1

In the process flow charts shown in FIGS. 1 and 2, the liquid mixture was partially collected through branched lines 36a, 36b, 36c and subjected to liquid chromatography analysis, and the results were compared with the results of NIR analysis, and are shown in Table 7.

In Table 7 below, Case 1 is calibration performed after introducing in a steady state, into the NIR analysis equipment, the reaction product (A), of which content was determined by liquid chromatography, and the sample (B), which was subjected to a spike test by adding a reference substance to the corresponding product. Case 2 is calibration performed using the reaction product (A) introduced in a steady state and the product (C) collected through real-time NIR analysis. Further, Case 3 is calibration performed using all samples A, B and C.

**[Table 7]**

| | Unit | Lactic acid | Lactic acid dimer | Acrylic acid | Acetaldehyde | Propanoic acid | 2,3-pentadione | Hydroxyacetone | Acetic acid |
|---|---|---|---|---|---|---|---|---|---|
| Case 1 | | 2.36 | 0.88 | 0.96 | 0.43 | 0.26 | 0.08 | 0.47 | 1.92 |
| Case 2 | wt% | 0.53 | 0.89 | 0.36 | 0.29 | 0.22 | 0.02 | 0.06 | 0.01 |
| Case 3 | | 0.53 | 0.79 | 0.30 | 0.14 | 0.27 | 0.04 | 0.06 | 0.19 |

As shown in Table 7, it was confirmed that the prediction error changed depending on the calibration curve learning method. Specifically, in Case 1, the measurement error increased up to 2.4% by weight, whereas when the product collected through real-time NIR analysis was used as in Case 2, the measurement error decreased to 1.0% by weight or less. As in Case 3, when calibration was performed using all samples, the maximum measurement error was maintained at 1.0% by weight or less, and the prediction errors were further reduced for major components such as lactic acids, acrylic acid, and acetaldehyde.

### [Reference numerals]

11: Gaseous feed (during reaction, lactic acid, steam, and carrier gas; during regeneration, oxygen and carrier gas)
12: Gaseous feed for reaction (lactic acid, steam, and carrier gas)
13: Gaseous feed for regeneration (oxygen and carrier gas)
2, 2a, 2b: Dehydration reactors
21: Gaseous product (during reaction, dehydration product; during regeneration, exhaust gas after regeneration)
22: Dehydration product
23: Exhaust gas after regeneration
3, 3a, 3b: Condensers
31: Liquid mixture
32, 34: Light products
33: High-boil-point liquid mixture
35: Low-boil-point liquid mixture
4: Near-infrared analysis signal processing and control device
41: Gaseous feed control (reaction/regeneration)
42: Reactor control
43: Condenser control
44a: Product signal (lactic acid, lactic acid oligomers, and propanoic acid)
44b: High-boil-point unreacted feed signal (lactic acid, lactic acid oligomers)
44c: Low-boil-point product signal (propanoic acid)

## Claims

1. A method of preparing an acrylic acid through lactic acid dehydration, the method comprising:
the step of preparing reaction products including the acrylic acid through the lactic acid dehydration in the presence of a catalyst (step 1);
the step of preparing a liquid mixture by condensing the reaction products (step 2);
the step of analyzing in real time the concentrations of lactic acid, lactic acid derivatives including lactic acid oligomers, acrylic acid, and propanoic acid in the liquid mixture using a near-infrared spectrometer by continuously passing the liquid mixture through an optical cell (step 3); and
the step of determining any one or more of the reaction temperature of the step 1 and regeneration of the catalyst, based on the analysis results of the step 3 (step 4).

2. The method of claim 1, wherein in the step 1, the lactic acid is supplied in the form of an aqueous solution, and a concentration of the lactic acid aqueous solution is 10% by weight to 80% by weight.

3. The method of claim 1, wherein the reaction of the step 1 is carried out under a temperature condition of 300°C to 500°C and a pressure condition of 1 atm to 5 atm.

4. The method of claim 1, wherein the preparation of the liquid mixture of the step 2 is carried out under a temperature condition of 50°C to 250°C.

5. The method of claim 1, wherein the preparation of the liquid mixture of the step 2 is carried out in a single condenser; or in a first condenser and a second condenser.

6. The method of claim 5, wherein the preparation of the liquid mixture of the step 2 is carried out in a first condenser and a second condenser,
the preparation of the liquid mixture in the first condenser is carried out under a temperature condition of 100°C to 250°C, and
the preparation of the liquid mixture in the second condenser is carried out under a temperature condition of 50°C to 150°C.

7. The method of claim 1, wherein the near-infrared ray used in the near-infrared spectrometer of the step 3 has a wavelength range of 4000 cm⁻¹ to 12500 cm⁻¹.

8. The method of claim 1, wherein as a result of the analysis of the step 3, when the concentration of lactic acid and lactic acid derivatives including lactic acid oligomers in the liquid mixture increases by 0.5% by weight to 10% by weight, as compared to the initial reaction conditions, the step of increasing the temperature of the reactor in the step 1 from 1°C to 50°C is included.

9. The method of claim 1, wherein as a result of the analysis in the step 3, when the concentration of propanoic acid in the liquid mixture is 0.1% by weight to 5% by weight, the catalyst regeneration step is performed.

10. The method of claim 9, wherein the catalyst regeneration step is performed using a method of removing coke by raising the temperature of the reactor to 320°C to 450°C and flowing a mixed gas containing oxygen at an oxygen concentration of 0.1 mol% to 20 mol%.

11. The method of claim 1, further comprising the step of aliquoting a portion of the liquid mixture to perform liquid chromatography analysis.

12. The method of claim 11, wherein the measurement errors of the lactic acid, lactic acid oligomer, acrylic acid, and propanoic acid concentrations in the liquid mixture, measured through the near-infrared spectrometry and liquid chromatography analysis, are 1.0% or less.

13. An apparatus for lactic acid dehydration, the apparatus comprising:
a reaction unit where lactic acid dehydration occurs in the presence of a catalyst;
a discharge unit discharging reaction products;
an absorption tower or condenser which is connected with the discharge unit to condense the reaction products, thereby preparing a liquid mixture;
a supply unit that continuously supplies the liquid mixture from the bottom of the absorption tower or condenser to an optical cell of a near-infrared spectrometer;
the near-infrared spectrometer that analyzes in real time the concentrations of lactic acid, lactic acid derivatives including lactic acid oligomers, acrylic acid, and propanoic acid in the supplied liquid mixture; and
a near-infrared analysis signal processing and control device.

14. The apparatus of claim 13, wherein the absorption tower or condenser is one or two.

15. The apparatus of claim 13, further comprising a liquid chromatography analyzer to which a part of the supply unit is branched and connected.
